Europäisches Patentamt

European Patent Office

Office européen des brevets

(19)

(11) Veröffentlichungsnummer: **0 028 760**

**A1**

(12) # EUROPÄISCHE PATENTANMELDUNG

(21) Anmeldenummer: **80106592.1**

(22) Anmeldetag: **25.10.80**

(51) Int. Cl.³: **C 07 C 21/20**
**C 07 C 17/34**
**//C07C69/743**

(30) Priorität: **12.11.79 DE 2945674**

(43) Veröffentlichungstag der Anmeldung:
**20.05.81 Patentblatt 81/20**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Heine, Hans-Georg, Dr.**
**Am Heckerhof 14**
**D-4150 Krefeld-1(DE)**

(72) Erfinder: **Hartmann, Willy, Dr.**
**Hohenzollernstrasse 25**
**D-4150 Krefeld-1(DE)**

(54) Verfahren zur Herstellung von 1,1-Dibrombutadien.

(57) Es wurde ein Verfahren zur Herstellung von 1,1 Dibrombutadien gefunden, daß dadurch gekennzeichnet ist, daß 4,4,4-Tribrombuten-1 mit einer organischen Base gegebenenfalls in einem Verdünnungsmittel in einem Temperaturbereich von 0 bis 100°C umgesetzt wird.

EP 0 028 760 A1

Croydon Printing Company Ltd.

BAYER AKTIENGESELLSCHAFT　　5090 Leverkusen, Bayerwerk
Zentralbereich　　　　　　　　Rt/Th-c
Patente, Marken und Lizenzen　Typ IVa-ZP


Verfahren zur Herstellung von 1.1-Dibrombutadien


Gegenstand der vorliegenden Erfindung ist ein Verfahren zur Herstellung von 1.1-Dibrombutadien.

1.1-Dibrombutadien entsteht als Nebenprodukt bei der Umsetzung von Allylbromid mit Bromoform und Natronlauge, in Gegenwart von Benzyltriethylammoniumchlorid (TEBA) (Chem. Commun. 1979, 210). Für die Herstellung präparativer Mengen 1.1-Dibrombutadien ist dieser Weg nicht geeignet.

Es wurde nun ein Verfahren zur Herstellung von 1.1-Dibrombutadien gefunden, das dadurch gekennzeichnet ist, daß 4.4.4-Tribrombuten-1 mit einer organischen Base gegebenenfalls in einem Verdünnungsmittel in einem Temperaturbereich von 0 bis 100° umgesetzt wird.

Das für die Herstellung des 1.1-Dibrombutadien benötigte 4.4.4-Tribrombuten-1 ist bekannt. Es kann nach bekannten Vorschriften hergestellt werden (sh. Chem. Commun. 1979, 210). Als organische Basen zur Durchführung des erfindungsgemäßen Verfahrens seien Alkalialkoholate, wie z.B. Natriummethylat, Natriumethylat, Natriumpropanolat; tert. Amine, wie z.B. Triethylamin,

BAD ORIGINAL

Dimethylanilin, Pyridin, Methyl-di-cyclohexylamin, Triethylendiamin (DABCO); Amidine, wie z.B. 1.5-Diaza-bicyclo/5.4.0/undecen-5 (DBU), 1.4-Diaza-bicyclo/4.3.0/ nonen-4 (DBN) genannt. Besonders bevorzugt sind Natriumethylat, Natriummethylat, DBU.

Bei der Verwendung der Alkoholate als Basen erfolgt unter den Reaktionsbedingungen keine weitere Elimi-nierung von Bromwasserstoff zu 1-Brom-butenin-1 und keine Substitution der vinylischen Bromatome durch Alkoxyreste unter Bildung von Ketenacetalen.

Als Verdünnungsmittel sind verschiedenartige, organische Lösungsmittel geeignet, wie z.B. Alkohole mit bis zu 10 C-Atomen, insbesondere dann, wenn Alkalialkoholate als Basen eingesetzt werden , gegebenenfalls substituierte Kohlenwasserstoffe, wie z.B. Benzol, Toluol, Petrolether, n-Hexan, Diphenylmethan, 1.2-Dichlorethan, Methylenchlorid; Ether, wie z.B. Tetrahydrofuran, 1.2-Dimethoxyethan, Di-ethylether, Dioxan, Bis-(2-methoxyethyl)-ether; aprotische dipolare Lösungsmittel, wie z.B. Dimethylformamid. Zweckmäßig wird ein solches Verdünnungsmittel in Kombination mit der verwendeten Base gewählt, das die Isolierung des 1.1-Dibrombutadien nicht - beispielsweise durch Azeotropbildung - erschwert.

Die Reaktionstemperatur liegt in einem Bereich von ca. 0-100°C, bevorzugt 0-50°C, besonders bevorzugt 20-50°C. Für viele Umsetzungen genügen 20°C oder eine Temperatur, die wenig darüber liegt. Die Reaktion wird bei Normal-druck durchgeführt.

Le A 20 023

Die Reaktionszeit liegt vorzugsweise zwischen einer halben und 5 Stunden. Sie kann aber auch bis zu 20 Stunden betragen, wenn relativ reaktionsträge tert. Amine als Basen verwendet werden.

Das Molverhältnis von Base zu 4.4.4-Tribrombuten-1 beträgt mindestens 0.9:1 und liegt vorzugsweise bei 1:1, höchstens bei 2:1.

Das erfindungsgemäße Verfahren wird in der Weise durchgeführt, daß man 4.4.4-Tribrombuten-1 gegebenenfalls in einem Verdünnungsmittel vorlegt und unter Rühren die Base gegebenenfalls in einem Verdünnungsmittel zutropft. Man kann aber auch in umgekehrter Reihenfolge die Reaktanden zusammenbringen, d.h. man legt die Base vor und tropft 4.4.4-Tribrombuten-1 unter Rühren zu. Im allgemeinen tritt eine exotherme Wärmetönung auf.

Oftmals ist die Umsetzung nach Zugabe der erforderlichen Menge Base beendet, so daß sich ein mehrstündiges Nachrühren des Reaktionsgemisches erübrigt. Das Aufarbeiten erfolgt in üblicher Weise. Die Reaktionslösung wird durch Zugabe von beispielsweise Salzsäure neutralisiert, die organische Phase mit einem üblichen organischen Lösungsmittel, wie z.B. Diethylether, Methylenchlorid, Toluol, verdünnt, getrocknet und eingedampft. Durch fraktionierendes Destillieren wird 1.1-Dibrombutadien isoliert. Das Aufarbeiten kann aber auch so erfolgen, daß aus dem Reaktionsansatz 1.1-Dibrombutadien direkt abdestilliert und das Rohdestillat gewaschen, getrocknet und rektifiziert wird. Base und Verdünnungsmittel sind hierbei so zu wählen, daß sie sich in leichter Weise vom Reaktionsprodukt abtrennen lassen.

Das nach dem erfindungsgemäßen Verfahren erhaltene 1.1-Dibrombutadien ist eine farblose Flüssigkeit vom Sdp. 43°/13 Torr. Sie verändert sich unter Licht- und Sauerstoffausschluß innerhalb von 6 Monaten nicht. Durch typische Radiakalstarter, wie Benzoylperoxid, wird 1.1-Dibrombutadien leicht polymerisiert. Dieser Prozeß wird durch Zugabe von Inhibitoren wie Hydrochinon unterdrückt. 1.1-Dibrombutadien verhält sich damit wie das homologe, 1.1-Dichlorbutadien (sh. US-Pat. 3 076 042).

Das nach dem erfindungsgemäßen Verfahren erhältliche 1.1-Dibrombutadien dient als wichtiges Zwischenprodukt für die Herstellung von Insektiziden (vgl. DE-OS 26 54 061). Umsetzung mit 1-Chlor-1-dimethylamino-2-methylpropen-1 in Gegenwart von Zinkchlorid liefert 2.2-Dimethyl-3-(ß.ß-dibromvinyl)-cyclobutanon, das nach $\alpha$-Bromierung mit Natriumalkoholat zum 2.2-Dimethyl-3-(ß.ß-dibromvinyl)-cyclopropancarbonsäureester umgesetzt wird. Verwendet man spezielle Alkohole, wie z.B. m-Phenoxybenzylalkohol, oder estert man mit solchen Alkoholen um, so gelangt man zu Verbindungen mit hoher insektizider Wirkung (Quart. Rev. 1979, 473).

Die folgenden Beispiele demonstrieren das erfindungsge-mäße Verfahren.

Le A 20 023

Beispiel 1

Durchgeführt nach dem in Chem. Commun. 1979, S. 210-211
angegebenen Verfahren:

29.3 g (0,1 mol) 4.4.4-Tribrombuten-1, 0.5 g Triethylbenzylammoniumchlorid und 40 ml 50 %ige Natronlauge
(0.75 mol) werden bei 30-40° 4 Stunden gerührt. Nach
dem Abkühlen des schwarzbraun gefärbten Reaktionsansatzes auf 20° setzt man 100 ml Petrolether (Siedebereich 40-60°) zu und rührt 15 Minuten intensiv. Man
trennt die organische Phase ab, trocknet und filtriert
sie. Destillieren unter Normaldruck hinterläßt keinen
Rückstand.

Extrahiert man ein in gleicher Weise erhaltenes Reaktionsgemisch statt mit Petrolether erschöpfend mit
Methylenchlorid, so erhält man nach Abtrennen, Trocknen
und Filtrieren der organischen Phase 7.4 g eines dunkel
gefärbten Öls, das laut NMR-Analyse 4.4.4-Tribrombuten-
1 und das gesuchte 1.1-Dibrombutadien enthält. Der Anteil an 1.1-Dibrombutadien im Rohprodukt ist wesentlich
kleiner als 10 %.

Beispiel 2

In einem mit Rührer, Tropftrichter und Rückflußkühler
versehenen 500 ml Rundkolben tropft man zu 58.6 g
(0.2 mol) 4.4.4-Tribrombuten-1 in 200 ml Pentan bei

Le A 20 023

20° innerhalb von 30 Minuten die Lösung von 30.4 g (0.2 mol) DBU in 100 ml Pentan. Nach 5 Stunden werden 100 ml Eiswasser uns 20 ml 1N HCl zugesetzt. Man trennt sie über wasserfreiem Natriumsulfat und engt sie unter Normaldruck ein. Fraktionierendes Destillieren an einer Spaltrohrkolonne liefert als Vorlauf 5.6 g vom Sdp. 38-43°/13 Torr 1,1-Dibrombutadien mit einem Gehalt von 76 % und eine Hauptfraktion von 25,3 g vom Sdp. 43°/13 Torr $n_D^{20}$ 1.5773 mit einem Gehalt $\geq$ 98 %.

(Gesamtausbeute 29,5 g = 70 %)

| $C_4H_4Br_2$ | Ber. | C 22.68 | H 1.90 | Br 75.42 |
|---|---|---|---|---|
| (211.9) | Gef. | 22.3 | 2.1 | 74.7 |

IR (CCl$_4$): 900 cm$^{-1}$. NMR (CDCl$_3$): $\delta$ 5.3 mc (H$_2$C=C, 2H), 6.4 mc (H$_2$C=CH, 1H) und 6.95 ppm mc (Br$_2$C=CH, 1H).

Beispiel 3

Zu der Lösung von 2.3 g (o.1 g-Atom) Natrium in 60 ml absol. Ethanol tropft man innerhalb von 15 Minuten unter Rühren bei 20° die Lösung von 29.3 g (o.1 mol) 4.4.4-Tribrombuten-1 in 30 ml absol. Ethanol. Man rührt 2 Stunden bei 40° nach, gibt das Reaktionsgemisch auf Eis und extrahiert mit Petrolether. Waschen des organischen Extrakts mit 50 ml Wasser, Trocknen über wasserfreiem Natriumsulfat, Filtrieren und Eindampfen liefern 17.2 g rohes 1.1Dibrombutadien. Fraktionierendes Destillieren ergibt 12.4 g 1.1Dibrombutadien sowie 2.3 g nicht umgesetztes 4.4.4-Tribrombuten-1.

Le A 20 023

Beispiel 4

Zu der Lösung von 12.4 g (0.1 mol) DBN in 100 ml Methylenchlorid tropft man unter Rühren bei 20° innerhalb 1 Stunde
die Lösung von 29.3 g (0.1 mol) 4.4.4-Tribrombuten-1
in 100 ml Methylenchlorid. Man rührt 1 Stunde bei 20°
nach, setzt 50 ml Eiswasser/1n HCl hinzu, trennt die
Phasen, trocknet die organische Phase und destilliert
fraktionierend. Man erhält 14.3 g 1.1-Dibrombutadien.

Beispiel 5

Zu der Lösung von 23.0 g (0.11 mol) 1.1-Dibrombutadien
in 200 ml absol. Methylenchlorid gibt man 18.0 g
(0.132 mol) trockenes Zinkchlorid und tropft anschließend
16.0 g (0.121 mol) 1-Chlor-1-dimethylamino-2-methyl-
propen-1 in 50 ml absol. Methylenchlorid innerhalb von
30 Minuten unter Rühren zu. Die Reaktionslösung erwärmt
sich hierbei von 22 auf 28°. Nach 7-stündigem Erhitzen
zum Rückfluß setzt man der Lösung 500 ml Wasser zu und
rührt über Nacht. Man trennt die Phasen, trocknet die
organische Phase über wasserfreiem Natriumsulfat,
filtriert und engt sie im Vakuum ein. Fraktionierendes
Destillieren liefert 22.5 g (73 %) 2.2-Dimethyl-3-
(ß.ß-dibromvinyl)-cyclobutanon vom Sdp. 80°/0.2 Torr
$n_D^{20}$ 1.5378, das in der Vorlage erstarrt. Schmp. 51-52°
(aus Methanol).

| $C_8H_{10}Br_2O$ | Ber. C 34.07 | H 3.58 | Br 56.68 |
|---|---|---|---|
| (282.0) | Gef. 33.8 | 3.78 | 57.2 |

IR $(CCl_4)$: 1800 $cm^{-1}$

Le A 20 023

- 8 -

Beispiel 6

Zu der Lösung von 20.8 g (0.074 mol) 2.2-Dimethyl-3-
(ß.ß-dibromvinyl)-cyclobutanon in 150 ml Essigsäureethylester werden bei 20° innerhalb von 30 Minuten
24.0 g (0.075 mol) Pyridiniumperbromid unter Rühren
gegeben. Man rührt 4 Stunden bei 20° nach, gibt das
Reaktionsgemisch auf Eis, trennt die Phasen, wäscht
die organische Phase neutral, trocknet sie über wasserfreiem Natriumsulfat und engt sie nach Filtrieren im
Vakuum ein. Den Rückstand (25.1 g, Gemisch der diastereomeren 4-Brom-2.2-dimethyl-3-(ß.ß-dibrombinyl)-cyclo-
butanone) löst man in 50 ml absol. Ethanol und tropft
bei 20° die Lösung von 2.0 g (0.09 g-Atom) Natrium in
100 ml absol. Ethanol unter Rühren zu. Nach 4-stündigem
Rühren bei 20° dampft man im Vakuum ein, nimmt den Rückstand in Methylenchlorid/1n HCl auf, trennt die Phasen,
wäscht die organische Phase neutral, trocknet, filtriert
und engt sie ein. Fraktionierendes Destillieren liefert
17.3 g einheitlichen 2.2-Dimethyl-3-(ß.ß-dibromvinyl)-
cyclopropancarbonsäureethylester vom Sdp. 80-87°C/0.1
bis 0.2 Torr $n_D^{20}$ 1.5115.

Le A 20 023

**0028760**

Patentansprüche

1.  Verfahren zur Herstellung von 1.1-Dibrombutadien, dadurch gekennzeichnet, daß 4.4.4-Tribrombuten-1 mit einer organischen Base gegebenenfalls in einem Verdünnungsmittel in einem Temperaturbereich von 0-100°C umgesetzt wird.

2.  Verfahren nach Anspruch 1, dadurch gekennzeichnet, daß die Base ein Alkalisalz eines Alkohols mit 1 bis 10 C-Atomen und das Verdünnungsmittel ein Alkohol mit 1 bis 10 C-Atomen ist, und der Temperaturbereich 20-50°C beträgt.

3.  Verfahren nach Anspruch 1, dadurch gekennzeichnet, daß die Base ein tert. Amin und das gegebenenfalls verwendete Verdünnungsmittel ein unter den Reaktionsbedingungen inertes organisches Lösungsmittel ist, und der Temperaturbereich 20-50°C beträgt.

4.  Verfahren nach Anspruch 1, dadurch gekennzeichnet, daß als Base ein cyclisches Amidin und als gegebenenfalls zu verwendendes Verdünnungsmittel ein aprotisches, unter den Reaktionsbedingungen stabiles organisches Lösungsmittel eingesetzt wird, und der Temperaturbereich 0-50°C beträgt.

5.  Verfahren nach einem der Ansprüche 1 bis 4, dadurch gekennzeichnet, daß das Molverhältnis von Base zu 4.4.4-Tribrombuten-1 0.9:1 bis 2:1 beträgt.

Le A 20 023

| | Europäisches<br>Patentamt | EUROPÄISCHER RECHERCHENBERICHT | Nummer der Anmeldung<br>EP 80 10 6592 |
|---|---|---|---|

<table>
<tr><th colspan="3">EINSCHLÄGIGE DOKUMENTE</th><th>KLASSIFIKATION DER<br>ANMELDUNG (Int Cl ?)</th></tr>
<tr><th>Kategorie</th><th>Kennzeichnung des Dokuments mit Angabe, soweit erforderlich, der<br>maßgeblichen Teile</th><th>betrifft<br>Anspruch</th><th rowspan="2">C 07 C 21/20<br>17/34//<br>C 07 C 69/743</th></tr>
<tr><td>AD</td><td>JOURNAL OF THE CHEMICAL SOCIETY,<br>Chemical Communications, 1. März<br>1979,<br>London, GB,<br>M.S. BAIRD et al.: "Reaction of<br>Allyl Bromides with Bromoform and<br>Base under Phase Transfer Condi-<br>tions", Seiten 210,211<br><br>   * Seite 211 *<br><br><br>    ----</td><td>1</td></tr>
<tr><td></td><td></td><td></td><td>RECHERCHIERTE<br>SACHGEBIETE (Int Cl ?)<br><br>C 07 C 17/34<br>21/20</td></tr>
<tr><td></td><td></td><td></td><td>KATEGORIE DER<br>GENANNTEN DOKUMENTE<br><br>X. von besonderer Bedeutung<br>A: technologischer Hintergrund<br>O. nichtschriftliche Offenbarung<br>P: Zwischenliteratur<br>T. der Erfindung zugrunde<br>  liegende Theorien oder<br>  Grundsatze<br>E: kollidierende Anmeldung<br>D: in der Anmeldung angeführtes<br>  Dokument<br>L: aus andern Gründen<br>  angeführtes Dokument</td></tr>
<tr><td colspan="3">Ⅹ   Der vorliegende Recherchenbericht wurde für alle Patentansprüche erstellt.</td><td>&: Mitglied der gleichen Patent-<br>familie   übereinstimmendes<br>Dokument</td></tr>
<tr><td>Recherchenort<br>  Den Haag</td><td colspan="2">Abschlußdatum der Recherche<br>  05-02-1981</td><td>Prüfer<br>  VAN GEYT</td></tr>
</table>

EPA form 1503.1 06.78